# EUROPÄISCHE PATENTSCHRIFT

(11) **EP 2 673 254 B1**
(45) Veröffentlichungstag und Bekanntmachung des Hinweises auf die Patenterteilung: **27.12.2017**
(21) Anmeldenummer: 12707243.7
(22) Anmeldetag: 03.03.2012
(51) Int. Cl.: C07C 67/03, C07C 69/52, C07C 69/587, C11B 3/14

(54) **EIN VERFAHREN ZUR DESTILLATION VON FETTSÄUREESTERN**
A PROCESS FOR THE DISTILLATION OF FATTY ACID ESTERS
PROCÉDÉ DE DISTILLATION DES ESTERS D'ACIDES GRAS

(30) Priorität: 08.03.2011 EP 11157277; 08.03.2011 US 201161450158 P
(43) Veröffentlichungstag der Anmeldung: 18.12.2013
(73) Patentinhaber: Cognis IP Management GmbH, 40589 Dusseldorf (DE)
(72) Erfinder: HORLACHER, Peter, 89287 Bellenberg (DE); HIETSCH, Dieter, 89257 Illertissen (DE)
(74) Vertreter: BASF IP Association
(86) Internationale Anmeldenummer: PCT/EP2012/000967
(87) Internationale Veröffentlichungsnummer: WO 2012/119745

(56) Entgegenhaltungen:
- EP-A2- 0 292 846
- WO-A1-87/03899
- WO-A1-2004/007655
- WO-A1-2004/043894
- US-A1- 2007 254 026
- ESTEBAN L ET AL: "Synthesis of 2-monoacylglycerols (2-MAG) by enzymatic alcoholysis of fish oils using different reactor types", BIOCHEMICAL ENGINEERING JOURNAL, ELSEVIER, AMSTERDAM, NL, Bd. 44, Nr. 2-3, 15. Mai 2009 (2009-05-15) , Seiten 271-279, XP026006770, ISSN: 1369-703X, DOI: DOI:10.1016/J.BEJ.2009.01.004 [gefunden am 2009-01-21]
- BEATE STOKKEREIT ET AL: 'EPAX 6000 EE product specification' INTERNET CITATION, [Online] 07 Februar 2013, Seiten 1 - 3, XP055360746 Gefunden im Internet: <URL:http://web.archive.org/web/20130207071 256/http://www.epax.com/kunder/epax/mm.nsf/ lupgraphics/Epax-6000EE.pdf/$file/Epax-6000 EE.pdf> [gefunden am 2017-03-31]

## Beschreibung

Die vorliegende Erfindung betrifft ein Verfahren zur Aufarbeitung eines Gemisches, welches Ester von EPA und/oder von DHA mit Monoalkoholen mit 1 bis 6 C-Atomen, Ester von anderen Fettsäuren mit Monoalkoholen mit 1 bis 6 C-Atomen und freies Cholesterin enthält, wobei das Verfahren das Hinzufügen eines Umesterungs-Katalysators zu dem Gemisch, die Überführung zumindest eines Teils des freien Cholesterins in verestertes Cholesterin und danach die Destillation des Gemisches umfasst, wobei die Destillation so ausgeführt wird, dass ein Produkt erhalten wird, welches EPA und/oder DHA, beide in Form ihrer Ester mit Monoalkoholen mit 1 bis 6 C-Atomen, in einer höheren relativen Menge, bezogen auf alle in dem Produkt enthaltenen Fettsäuren in freier oder gebundener Form, enthält als das genannte Gemisch. Weiterhin betrifft die vorliegende Erfindung ein Produkt, welches erhältlich ist nach dem erfindungsgemäßen Verfahren, wobei das Produkt 10 bis 99,99 Gew.-% Ester von EPA und/oder von DHA mit Monoalkoholen mit 1 bis 6 C-Atomen und 0,0001 bis maximal 0,3 Gew.-% Cholesterin in freier oder gebundener Form enthält.

Eicosapentaensäure (EPA) und Docosahexaensäure (DHA) werden in Nahrungsergänzungsmitteln und in funktionellen Lebensmitteln (so genanntes functional food) verwendet. EPA und DHA können zum Beispiel aus Fischöl gewonnen werden, in dem beide in Form von Triglyceriden vorkommen.

Um EPA und DHA aus Fischöl anzureichern kann das Fischöl, welches überwiegend aus Fettsäuretriglyceriden besteht, zu Fettsäureethylestern umgeestert werden. Die Fettsäureethylester lassen sich dann destillativ trennen. So können EPA-Ethylester und DHA-Ethylester angereichert werden. Das ist bekannt. Es ist dem Fachmann auch bekannt, wie die Destillation zu gestalten ist, um eine Anreicherung der Ethylester von EPA und/oder DHA im Vergleich zu den Ethylestern anderer Fettsäuren zu erreichen. Zum Beispiel kann eine zweistufige Destillation angewandt werden, wobei in einer ersten Stufe leichtflüchtige unerwünschte Komponenten abdestilliert werden und die Ethylester von EPA und/oder DHA im Destillationssumpf verbleiben. In einer zweiten Stufe können dann bei stärkerem Vakuum und/oder höherer Temperatur die Ethylester von EPA und/oder DHA abdestilliert werden, wobei unerwünschte Komponenten im Destillationssumpf verbleiben.

Hierbei können zwei Probleme auftreten. Zum einen enthält Fischöl unerwünschtes Cholesterol (auch Cholesterin genannt). Dieses liegt zum Teil in freier Form vor, zum Teil liegt es als Ester mit Fettsäuren, also als Cholesterinester, vor. Das freie Cholesterin hat einen Siedepunkt, der den Siedepunkten von EPA-Ethylester und von DHA-Ethylester ähnlich ist. Deshalb verbleibt bei der destillativen Anreicherung von EPA-Ethylester und von DHA-Ethylester freies Cholesterin in den gewünschten Estern. Dies ist u. a. deshalb unerwünscht, weil Cholesterin vielfach als in Nahrungsmitteln unerwünscht betrachtet wird.

WO 2004/007655 offenbart ein Verfahren zur Reduktion des Gehaltes an freiem Cholesterin in Fischöl durch Schleppmitteldestillation. Dieses Verfahren reduziert allerdings nicht den Gehalt an höhersiedendem Cholesterinester in Fischöl. D. h., auch wenn der Gehalt an freiem Cholesterin in Fischöl reduziert wurde, bevor dieses zu Ethylestern umgeestert wird, aus denen dann EPA-Ethylester und DHA-Ethylester destillativ angereichert werden können, verbleibt Cholesterinester in dem Fischöl, das umgeestert werden soll. Bei der Umesterung des Fischöls zu Ethylestern wird nun aus den Cholesterinestern wieder freies Cholesterin freigesetzt, so dass bei der Destillation der Ethylester das Problem bestehen bleibt, dass die erhaltenen Ester von EPA und DHA unerwünscht hohe Mengen an Cholesterin enthalten. US2007/254026 A1 verwendet "EPAX 6000 EE" als Einsatzstoff und belegt damit dessen Verfügbarkeit zum Zeitpunkt der Anmeldung dieser US-Schrift. EPAX 6000 EE ist ein Omega-3-haltiger Ester, enthaltend inter alia 500 mg/g EPA und DHA als Ethylester sowie 1 mg/g Cholesterin.

Die entsprechende Produktbeschreibung kann im Internet unter dem URL "https:// web-beta.archive.org/web/*/http:/www.epax.com/kunder/epax/mm.nsf/lupgraphics/ Epax-6000EE.pdf/*" innerhalb der "Wayback-Machine" gefunden werden (Suchbegriff "http:/www.epax.com/kunder/epax/mm.nsf/lupgraphics/Epax-6000EE.pdf").

Außerdem führt die Umesterung des Fischöls zu Ethylestern in der Regel nicht zu einer vollständigen Überführung der Fettsäuren aus der Triglyceridform in die Ethylesterform. Ein Teil der Fettsäuren bleibt in Form von Monoglyceriden an Glycerin gebunden. Auch diese Monoglyceride stellen ein Problem für die destillative Anreicherung von EPA-Ethylester und von DHA-Ethylester dar. Bestimmte Fettsäure-Monoglyceride, z. B. diejenigen der Hexadecansäure haben einen Siedepunkt, der den Siedepunkten von EPA-Ethylester und von DHA-Ethylester ähnlich ist. Deshalb verbleiben diese Monoglyceride bei der destillativen Anreicherung von EPA-Ethylester und von DHA-Ethylester in dem Produkt, welches EPA-Ethylester und DHA-Ethylester in angereicherter Menge enthält. Auch dies ist unerwünscht. Ggf. vorhandene unerwünschte Monoglyceride können nachträglich durch Auskristallisieren bei niedrigen Temperaturen (so genannte Winterisierung) zum Teil entfernt werden.

Die in den vorausgegangenen Absätzen beschriebenen Probleme treten nicht nur bei den Ethylestern von EPA und von DHA auf, sondern auch bei deren Ester mit anderen Monoalkoholen mit 1 bis 6 C-Atomen, da diese ähnliche Siedepunkte haben.

Der vorliegenden Erfindung liegt deshalb die Aufgabe zu Grunde, ein Verfahren bereit zu stellen, das es erlaubt, ein Gemisch von Estern von Fettsäuren mit Monoalkoholen mit 1 bis 6 C-Atomen, welches freies Cholesterin enthält, und welches die Ester von EPA und/oder DHA mit Monoalkoholen mit 1 bis 6 C-Atomen enthält, aufzuarbeiten, so dass der Anteil von EPA und/oder DHA im erhaltenen Produkt gegenüber dem genannten Gemisch erhöht wird, wobei der Gehalt an freiem Cholesterin im erhaltenen Produkt geringer sein soll als in dem genannten Gemisch.

Diese Aufgabe wird gelöst durch das Verfahren gemäß den Ansprüchen der vorliegenden Erfindung.

Monoalkoholen mit 1 bis 6 C-Atomen sind erfindungsgemäß insbesondere Methanol, Ethanol, Propanol, n-Butanol, n-Pentanol und n-Hexanol.

Ein Umesterungs-Katalysator ist erfindungsgemäß jeder Katalysator, der die Umesterung eines Esters einer Carbonsäure mit einem ersten Alkohol in Anwesenheit eines zweiten Alkohols zu einem Ester der Carbonsäure mit dem zweiten Alkohol unter Freisetzung des ersten Alkohols katalysiert. Insbesondere sind hierzu nennen Lipasen, Alkalisalze von Alkanolen, z. B. Natriumethanolat (auch Natriumethylat genannt).

Weiterhin liegt der vorliegenden Erfindung in einer bevorzugten Ausführungsform die Aufgabe zu Grunde, ein Verfahren bereit zu stellen, bei dem der Gehalt an außerdem in dem Gemisch ggf. vorhandenen Monoglyceriden im erhaltenen Produkt geringer sein soll als in dem genannten Gemisch.

Diese Aufgabe wird gelöst durch das erfindungsgemäße Verfahren, welches in seiner allgemeinen Form in der folgenden, ersten Ausführungsform wiedergegeben ist.
1. Ein Verfahren zur Aufarbeitung eines Gemisches, welches enthält Ester von EPA und/oder von DHA mit Monoalkoholen mit 1 bis 6 C-Atomen, Ester von anderen Fettsäuren mit Monoalkoholen mit 1 bis 6 C-Atomen und freies Cholesterin, wobei das Verfahren umfasst das Hinzufügen eines Umesterungs-Katalysators zu dem Gemisch, die Überführung zumindest eines Teils des freien Cholesterins in verestertes Cholesterin und danach die Destillation des Gemisches, wobei die Destillation so ausgeführt wird, dass ein Produkt erhalten wird, welches EPA und/oder DHA, beide in Form ihrer Ester mit Monoalkoholen mit 1 bis 6 C-Atomen, in einer höheren relativen Menge, bezogen auf alle in dem Produkt enthaltenen Fettsäuren in freier oder gebundener Form, enthält als das genannte Gemisch.

Bevorzugte Ausführungsformen des erfindungsgemäßen Verfahrens werden durch die folgenden Ausführungsformen wiedergegeben.
2. Das Verfahren nach Ausführungsform 1, wobei es sich bei den Monoalkoholen mit 1 bis 6 C-Atomen um Ethanol handelt.
3. Das Verfahren nach einer der Ausführungsformen 1 oder 2, wobei der Umesterungs-Katalysator ausgewählt wird aus der Gruppe bestehend aus einer Lipase, einem Alkalisalz eines Monoalkohols mit 1 bis 6 C-Atomen und Natriumethanolat.
4. Das Verfahren nach einer der Ausführungsformen 1 bis 3, wobei das Gemisch aus einem Fischöl, bevorzugt durch Umesterung mit einem Monoalkohol mit 1 bis 6 C-Atomen (bevorzugt mit Ethanol), erhalten wird.
5. Das Verfahren nach einer der Ausführungsformen 1 bis 4, wobei das Gemisch enthält 10 bis 40 Gew.-% Ester von EPA und/oder von DHA mit Monoalkoholen mit 1 bis 6 C-Atomen, 50 bis 90 Gew.-% Ester von anderen Fettsäuren mit Monoalkoholen mit 1 bis 6 C-Atomen und 0,1 bis 1 Gew.-% freies Cholesterin
6. Das Verfahren nach einer der Ausführungsformen 1 bis 5, wobei zwischen der Überführung zumindest eines Teils des freien Cholesterins in verestertes Cholesterin und der Destillation des Gemisches ein Wasch-Schritt, bevorzugt mit Wasser, und optional danach ein Trocknungsschritt, bevorzugt durch Trocknung im Vakuum, erfolgt.
7. Das Verfahren nach einer der Ausführungsformen 1 bis 6, wobei die Destillation des Gemisches in zwei Stufen erfolgt, wobei in einer ersten Stufe EPA und/oder DHA, beide in Form ihrer Ester mit Monoalkoholen mit 1 bis 6 C-Atomen, als Sumpfprodukt erhalten werden, und wobei in einer zweiten Stufe das Sumpfprodukt einer Destillation unterworfen wird, bei der EPA und/oder DHA, beide in Form ihrer Ester mit Monoalkoholen mit 1 bis 6 C-Atomen, als Kopfprodukt erhalten werden.
8. Das Verfahren nach einer der Ausführungsformen 1 bis 7, wobei das Gemisch zusätzlich enthält Monoglyceride von Fettsäuren, insbesondere Monoglyceride von Fettsäuren, wobei die Fettsäuren 14 bis 18, insbesondere 16, C-Atome haben, und wobei diese Monoglyceride bevorzugt in einer Menge von 1 bis 10 Gew.-% in dem Gemisch vorhanden sind.

Weiterhin liegt der vorliegenden Erfindung die Aufgabe zu Grunde, ein Gemisch von Estern von Fettsäuren mit Monoalkoholen mit 1 bis 6 C-Atomen, welches die Ester von EPA und/oder DHA mit Monoalkoholen mit 1 bis 6 C-Atomen enthält, bereit zu stellen, welches zwar Cholesterin, in freier oder in gebundener Form, enthält, aber nur in geringer Menge.

Weiterhin liegt der vorliegenden Erfindung in einer bevorzugten Ausführungsform die Aufgabe zu Grunde, ein Gemisch von Estern von Fettsäuren mit Monoalkoholen mit 1 bis 6 C-Atomen, welches die Ester von EPA und/oder DHA mit Monoalkoholen mit 1 bis 6 C-Atomen enthält, bereit zu stellen, welches zwar freies Cholesterin enthält, aber nur in geringer Menge, wobei das Gemisch außerdem zwar Monoglyceride enthält aber diese ebenfalls nur in geringer Menge.

Diese Aufgabe wird gelöst durch ein Produkt, welches erhältlich ist nach dem Verfahren gemäß einem der Ausführungsformen 1 - 8, wobei das Produkt enthält 30 bis 99 Gew.-% Ester von EPA und/oder von DHA mit Monoalkoholen mit 1 bis 6 C-Atomen, bevorzugt mit Ethanol, und 0,0001 bis maximal 0,3 Gew.-%, insbesondere 0,001 bis maximal 0,1 Gew.-%, insbesondere 0,002 bis maximal 0,05 Gew.-%, insbesondere 0,002 bis maximal 0,01 Gew.-%, Cholesterin in freier oder gebundener Form, wobei dieses Produkt zusätzlich enthält 0,01 bis 5 Gew.-%, insbesondere 0,02 bis 4 Gew.-%, insbesondere 0,03 bis 3 Gew.-%, insbesondere 0,05 bis 1 Gew.-% Monoglyceride von Fettsäuren, insbesondere Monoglyceride von Fettsäuren, wobei die Fettsäuren 14 bis 18, insbesondere 16, C-Atome haben.

### Beispiele

### %-Angaben bedeuten Gew.-%, wenn nichts anderes angegeben ist.

### Beispiel 1 (Vergleichsbeispiel)

### Umesterung

400,0 g Fischöl 20/10 (Fischöl mit 20 % EPA und 10 % DHA bezogen auf alle Fettsäuren) (Gesamtcholesterolgehalt: 0,36 Gew.-%) wurden in einem 2 I-Dreihalskolben vorgelegt und mit Stickstoff inertisiert. Anschließend wurden 120,0 g Ethanol absolut und 12,5 g Natriumethylat (Natriumethoxid) (20 Gew.-% in Ethanol) hinzugeben, das Reaktionsgemisch aufgeheizt und 2 Stunden unter Rückfluss reagieren lassen. Das überschüssige Ethanol wurde abdestilliert, anschließend wurde das Reaktionsgemisch zur Phasentrennung eine Stunde bei 75°C stehen gelassen. Die Unterphase (Glycerinphase) wurde abgetrennt und die Oberphase 3 mal mit 100 g warmem Wasser bei 75°C gewaschen. Nach dem Abtrennen des letzten Waschwassers wurde der Ansatz im Vakuum (25 mbar) bei 90°C getrocknet. Auswaage: 416,0 g Fischöl-Ethylester (Fischöl-EE) 20/10, roh (klar, orangefarben).

Zusammensetzung des Fischöl-Ethylesters (in GC-Flächen-%):

| | |
|---|---|
| Monoglycerid | 5,8 |
| Diglycerid | 2,8 |
| Triglycerid | n.n. |
| Ethylester | 91,4 |

Die Aufkonzentrierung der EPA/DHA-EE im rohen Fischöl-EE 20/10 wurde nach bekannter Art und Weise wie im Folgenden beschrieben durch Destillation vorgenommen.

### Destillation 1

416 g Fischöl-EE 20/10 roh wurden mittels Laborkurzwegdestillation (KD) unter folgenden Bedingungen destilliert:
Öltemperatur 95°C/ Druck 0,02 mbar/ Einspeisung 3,8 g pro Min./ Entgaser 75°C/
Innenkühler 40°C
Destillat: 245,4 g (fast farblos, klar, 59 %)
Rückstand: 170,6 g (orange, klar, 41 %)

### Destillation 2

Der Rückstand aus der Destillation 1 wurde nochmals mittels KD destilliert:
Öltemperatur 180°C/ Druck 0,015 mbar/ Einspeisung 2,7 g pro Min./ Entgaser 75°C/
Innenkühler 40°C
Rückstand: 13,7 g (rot-orange, fast klar, 8 %).
Destillat: 156,9 g (hellgelb, klar, 92 %).

### Abtrennung von Unlöslichem

Zur Abtrennung der bei 0°C unlöslichen Bestandteile wurde das Ethylesterkonzentrat (Destillat der Destillation 2) über Nacht bei 0°C gelagert und am nächsten Morgen mittels einer Labornutsche, die mit der Filterplatte Beco CP1 KS 4L bestückt war, filtriert.
Filtrat: 140,0 g
Filterkuchen: 16,9 g (10,8%)
Zusammensetzung des Filtrats:

| | |
|---|---|
| Monoglycerid (GC-Flächen-%) | 10,0 |
| Ethylester (GC-Flächen-%) | 90,0 |
| Cholesterol (Gew.-%) | 0,34 |

Wie der Tabelle zu entnehmen ist, entsprach der Gesamtcholesteringehalt nahezu dem Ausgangswert von 0,36 Gew.-%.

### Beispiel 2 (erfindungsgemäß)

### Umesterung

400,0 g Fischöl 20/10 (Gesamtcholesterolgehalt: 0,36 Gew.-%) wurden in einem 2 I-Dreihalskolben vorgelegt und mit Stickstoff inertisiert. Anschließend wurden 120,0 g Ethanol absolut und 12,5 g Na-Ethylat (20 %ig) hinzugeben, das Reaktionsgemisch aufgeheizt und 2 Stunden unter Rückfluss reagieren lassen. Das überschüssige Ethanol wurde abdestilliert und anschließend zur Phasentrennung 1 Stunde bei 75°C stehen gelassen. Die Unterphase (Glycerinphase) wurde abgetrennt. Zu dem Rückstand wurden 2,5 g Na-Ethylat (20 %ig) hinzugeben. Nun wurde Vakuum (25 mbar) angelegt und der Ansatz 2 Stunden bei 110°C gerührt. Anschließend wurde abgekühlt auf ca. 80°C und Normaldruck mittels Stickstoff hergestellt. Daraufhin wurde der Ansatz wie im Beispiel 1 beschrieben 3 mal mit Wasser gewaschen und aufgearbeitet. Auswaage: 410,6 g Fischöl-EE 20/10, roh (klar, orangefarben). Zusammensetzung des Fischöl-Ethylesters (in GC-Flächen-%):

| | |
|---|---|
| Monoglycerid | 2,9 |
| Diglycerid | 2,2 |
| Triglycerid | 14,3 |
| Ethylester | 80,6 |

Die Aufkonzentrierung der EPA/DHA-EE im rohen Fischöl-EE 20/10 wurde analog Beispiel 1 durchgeführt. Zur Abtrennung von bei 0°C unlöslichen Bestandteilen wurde das Produkt (147,8 g) über Nacht bei 0°C gelagert. Da sich kein Niederschlag gebildet hatte, entfiel die Filtration und damit fiel auch kein Filterkuchen an.

### Zusammensetzung des Produkts:

| | |
|---|---|
| Monoglycerid (GC-Flächen-%) | 4,8 |
| Ethylester (GC-Flächen-%) | 95,2 |
| Cholesterol (Gew.-%) | <0,01 |

Wie der Tabelle zu entnehmen ist, wurde bei Zusatz von Katalysator (Natriumethylat) zur Nachreaktion der Gesamtcholesteringehalt um mehr als 95% reduziert.

### Beispiel 3 (erfindungsgemäß):

450 g Fischöl-EE 20/10 (5,7 % Mononglycerid, 2,2 % Diglycerid, 0,2 % Gew.-% Gesamtcholesterin) wurden in einem 1 Liter Dreihalskolben vorgelegt und mit Stickstoff inertisiert. Hierzu wurden 9,0 g Novozym® 435 NG (ein Enzymimmobilisat einer Lipase) geben. Der Ansatz wurde auf 77°C aufgeheizt und ein Vakuum von 1 mbar angelegt. Nach 48 Stunden Reaktionszeit wurde abgekühlt und das Enzymimmobilisat abfiltriert. Auswaage: 421 g

Die Aufkonzentrierung der EPA/DHA-EE im rohen Fischöl-EE 20/10 wurde analog Beispiel 1 durchgeführt. Zur Abtrennung von bei 0°C unlöslichen Bestandteilen wurde das Produkt (121,8 g) über Nacht bei 0°C gelagert. Da sich kein Niederschlag gebildet hatte, entfiel die Filtration und damit fiel auch kein Filterkuchen an.

Zusammensetzung des Produkts:

| | |
|---|---|
| Monoglycerid (GC-Flächen-%) | 0,1 |
| Ethylester (GC-Flächen-%) | 99,9 |
| Cholesterol (Gew.-%) | 0,01 |

## Patentansprüche

1. Ein Verfahren zur Aufarbeitung eines Gemisches, welches enthält
• Ester von EPA und/oder von DHA mit Monoalkoholen mit 1 bis 6 C-Atomen,
• Ester von anderen Fettsäuren mit Monoalkoholen mit 1 bis 6 C-Atomen und
• freies Cholesterin,
wobei das Verfahren umfasst
• das Hinzufügen eines Umesterungs-Katalysators zu dem Gemisch,
• die Überführung zumindest eines Teils des freien Cholesterins in verestertes Cholesterin und danach
• die Destillation des Gemisches,
wobei die Destillation so ausgeführt wird, dass ein Produkt erhalten wird, welches EPA und/oder DHA, beide in Form ihrer Ester mit Monoalkoholen mit 1 bis 6 C-Atomen, in einer höheren relativen Menge, bezogen auf alle in dem Produkt enthaltenen Fettsäuren in freier oder gebundener Form, enthält als das genannte Gemisch.

2. Das Verfahren nach Anspruch 1, wobei der
Umesterungs-Katalysator ausgewählt wird aus der Gruppe bestehend aus einer Lipase, einem Alkalisalz eines Monoalkohols mit 1 bis 6 C-Atomen und
Natriumethanolat.

3. Das Verfahren nach einem der Ansprüche 1 bis 2, wobei das Gemisch aus einem Fischöl durch Umesterung mit einem Monoalkohol mit 1 bis 6 C-Atomen
(bevorzugt mit Ethanol), erhalten wird.

4. Das Verfahren nach einem der Ansprüche 1 bis 3, wobei das Gemisch enthält
• 10 bis 40 Gew.-% Ester von EPA und/oder von DHA mit Monoalkoholen mit 1 bis 6 C-Atomen,
• 50 bis 90 Gew.-% Ester von anderen Fettsäuren mit Monoalkoholen mit 1 bis 6 C-Atomen und
• 0,1 bis 1 Gew.-% freies Cholesterin.

5. Das Verfahren nach einem der Ansprüche 1 bis 4, wobei zwischen der Überführung zumindest eines Teils des freien Cholesterins in verestertes Cholesterin und der Destillation des Gemisches ein Wasch-Schritt mit Wasser und danach ein Trocknungsschritt durch Trocknung im Vakuum erfolgt.

6. Das Verfahren nach einem der Ansprüche 1 bis 5, wobei die Destillation des Gemisches in zwei Stufen erfolgt,
wobei in einer ersten Stufe EPA und/oder DHA, beide in Form ihrer Ester mit Monoalkoholen mit 1 bis 6 C-Atomen, als Sumpfprodukt erhalten werden, und wobei in einer zweiten Stufe das Sumpfprodukt einer Destillation unterworfen wird, bei der EPA und/oder DHA, beide in Form ihrer Ester mit Monoalkoholen mit 1 bis 6 C-Atomen, als Kopfprodukt erhalten werden.

7. Das Verfahren nach einem der Ansprüche 1 bis 6, wobei das Gemisch zusätzlich enthält
• Monoglyceride von Fettsäuren.

8. Ein Produkt, welches erhältlich ist nach dem Verfahren gemäß einem der Ansprüche 1 bis 7, wobei das Produkt enthält
• 30 bis 99,9 Gew.-% Ester von EPA und/oder von DHA mit Monoalkoholen mit 1 bis 6 C-Atomen, bevorzugt mit Ethanol,
• 0,0001 bis maximal 0,3 Gew.-% Cholesterin in freier oder gebundener Form, und
• 0,01 bis 5 Gew.-% Monoglyceride von Fettsäuren, bevorzugt von Fettsäuren mit 16 C-Atomen.

## Claims

1. A method for working up a mixture which comprises
• esters of EPA and/or DHA with monohydric alcohols having 1 to 6 carbon atoms,
• esters of other fatty acids with monohydric alcohols having 1 to 6 carbon atoms and
• free cholesterol,
wherein the method comprises
• adding a transesterification catalyst to the mixture,
• converting at least some of the free cholesterol into esterified cholesterol and thereafter
• distilling the mixture,
wherein the distillation is carried out in such a manner that a product is obtained which contains EPA and/or DHA, both in the form of esters thereof with monohydric alcohols having 1 to 6 carbon atoms, in a higher relative amount, based on all fatty acids in free or bound form present in the product, than said mixture.

2. The method according to claim 1, wherein the transesterification catalyst is selected from the group consisting of a lipase, an alkali metal salt of a monohydric alcohol having 1 to 6 carbon atoms and sodium ethanolate.

3. The method according to any one of claims 1 to 2, wherein the mixture is obtained from a fish oil by transesterification with a monohydric alcohol having 1 to 6 carbon atoms (preferably with ethanol).

4. The method according to any one of claims 1 to 3, wherein the mixture contains
• 10 to 40% by weight esters of EPA and/or DHA with monohydric alcohols having 1 to 6 carbon atoms,
• 50 to 90% by weight esters of other fatty acids with monohydric alcohols having 1 to 6 carbon atoms and
• 0.1 to 1% by weight free cholesterol.

5. The method according to any one of claims 1 to 4, wherein a washing step with water and thereafter a drying step by drying in vacuum proceeds between the conversion of at least some of the free cholesterol into esterified cholesterol and the distillation of the mixture.

6. The method according to any one of claims 1 to 5, wherein the mixture is distilled in two stages,
wherein, in a first stage, EPA and/or DHA, both in the form of esters thereof with monohydric alcohols having 1 to 6 carbon atoms, are obtained as bottom-phase product, and wherein, in a second stage, the bottom-phase product is subjected to a distillation, in which EPA and/or DHA, both in the form of esters thereof with monohydric alcohols having 1 to 6 carbon atoms, are obtained as overhead product.

7. The method according to any one of claims 1 to 6, wherein the mixture additionally comprises
• monoglycerides of fatty acids.

8. A product which is obtainable by the method according to any one of claims 1 to 7, wherein the product contains
• 30 to 99.9% by weight esters of EPA and/or DHA with monohydric alcohols having 1 to 6 carbon atoms, preferably with ethanol,
• 0.0001 to a maximum of 0.3% by weight cholesterol in free or bound form, and
• 0.01 to 5% by weight monoglycerides of fatty acids, preferably of fatty acids having 16 carbon atoms.

## Revendications

1. Procédé de traitement d'un mélange, qui contient :
- des esters d'EPA et/ou de DHA avec des monoalcools de 1 à 6 atomes C,
- des esters d'autres acides gras avec des monoalcools de 1 à 6 atomes C, et
- du cholestérol libre,
le procédé comprenant :
- l'ajout d'un catalyseur de transestérification au mélange,
- la transformation d'au moins une partie du cholestérol libre en cholestérol estérifié, puis
- la distillation du mélange,
la distillation étant réalisée de manière à obtenir un produit qui contient de l'EPA et/ou du DHA, tous les deux sous la forme de leurs esters avec des monoalcools de 1 à 6 atomes C, en une quantité relative plus élevée, par rapport à tous les acides gras contenus dans le produit sous forme libre ou reliée, que le mélange mentionné.

2. Procédé selon la revendication 1, dans lequel le catalyseur de transestérification est choisi dans le groupe constitué par une lipase, un sel alcalin d'un monoalcool de 1 à 6 atomes C et l'éthanolate de sodium.

3. Procédé selon l'une quelconque des revendications 1 à 2, dans lequel le mélange est obtenu à partir d'une huile de poisson par transestérification avec un monoalcool de 1 à 6 atomes C (de préférence avec de l'éthanol).

4. Procédé selon l'une quelconque des revendications 1 à 3, dans lequel le mélange contient :
- 10 à 40 % en poids d'esters d'EPA et/ou de DHA avec des monoalcools de 1 à 6 atomes C,
- 50 à 90 % en poids d'esters d'autres acides gras avec des monoalcools de 1 à 6 atomes C, et
- 0,1 à 1 % en poids de cholestérol libre.

5. Procédé selon l'une quelconque des revendications 1 à 4, dans lequel une étape de lavage avec de l'eau, puis une étape de séchage par séchage sous vide ont lieu entre la transformation d'au moins une partie du cholestérol libre en cholestérol estérifié et la distillation du mélange.

6. Procédé selon l'une quelconque des revendications 1 à 5, dans lequel la distillation du mélange a lieu en deux étapes, selon lesquelles
lors d'une première étape, l'EPA et/ou le DHA, tous les deux sous la forme de leurs esters avec des monoalcools de 1 à 6 atomes C, sont obtenus en tant que produit de fond et, lors d'une seconde étape, le produit de fond est soumis à une distillation lors de laquelle l'EPA et/ou le DHA, tous les deux sous la forme de leurs esters avec des monoalcools de 1 à 6 atomes C, sont obtenus en tant que produit de tête.

7. Procédé selon l'une quelconque des revendications 1 à 6, dans lequel le mélange contient en outre :
- des monoglycérides d'acides gras.

8. Produit, qui peut être obtenu par le procédé selon l'une quelconque des revendications 1 à 7, le produit contenant :
- 30 à 99,9 % en poids d'esters d'EPA et/ou de DHA avec des monoalcools de 1 à 6 atomes C, de préférence avec de l'éthanol,
- 0,0001 à au plus 0,3 % en poids de cholestérol sous forme libre ou reliée, et
- 0,01 à 5 % en poids de monoglycérides d'acides gras, de préférence d'acides gras de 16 atomes C.
